Europäisches Patentamt

European Patent Office

Office européen des brevets

(19)

(11) Veröffentlichungsnummer: **0 118 019 B2**

(12) **NEUE EUROPÄISCHE PATENTSCHRIFT**

(45) Veröffentlichungstag der neuen Patentschrift: 09.05.90

(51) Int. Cl.⁵: **A 23 F 5/20**

(21) Anmeldenummer: **84100954.1**

(22) Anmeldetag: **31.01.84**

(54) Verfahren zur Entcoffeinierung von Röstkaffee.

(30) Priorität: **03.02.83 DE 3303679**

(43) Veröffentlichungstag der Anmeldung:
**12.09.84 Patentblatt 84/37**

(45) Bekanntmachung des Hinweises auf die Patenterteilung:
**28.05.86 Patenblatt 86/22**

(45) Bekanntmachung des Hinweises auf die Entscheidung über den Einspruch:
**09.05.90 Patentblatt 90/19**

(84) Benannte Vertragsstaaten:
**AT BE CH DE FR GB IT LI NL SE**

(56) Entgegenhaltungen:
**DE-C-2 634 535**
**DE-C-2 637 197**
**FR-A-2 176 008**
**FR-A-2 361 824**
**FR-A-2 408 607**
**US-A-3 108 876**
**US-A-3 477 856**

(73) Patentinhaber: **HAG GF AKTIENGESELLSCHAFT**
**Hagstrasse 3**
**D-2800 Bremen 1 (DE)**

(72) Erfinder: **Gehrig, Manfred, Dr.**
**Lug-ins-Land 10**
**D-8069 Wolnzach (DE)**
Erfinder: **Vitzthum, Otto, Dr.**
**Upper Borg 170**
**D-2800 Bremen (DE)**
Erfinder: **Wienges, Hans**
**Helene-Neesen-Strasse 8**
**D-2800 Bremen (DE)**

(74) Vertreter: **Abitz, Walter, Dr.-Ing. et al**
**Abitz, Morf, Gritschneder, Freiherr von**
**Wittgenstein Postfach 86 01 09**
**D-8000 München 86 (DE)**

EP 0 118 019 B2

Courier Press, Leamington Spa, England.

## Beschreibung

Die Erfindung betrifft ein Verfahren zum Extrahieren von Coffein aus gemahlenem Röstkaffee mit $CO_2$ bei einem Druck von 50 bis 200 bar, wobei das mit Coffein beladene $CO_2$ zur Abtrennung des Coffeins durch einen sauren Ionenaustauscher geführt wird.

Die Verwendung von Kohlendioxid zur Entfernung von Coffein aus Kaffee ist bekannt. Es sind zahlreiche derartige Entcoffeinierungsverfahren in der Literatur beschreiben worden.

In der DE—PS 20 05 293 wird die Entcoffeinierung von angefeuchteten Rohkaffeebohnen mit überkritischem $CO_2$, d. h. bei Temperaturen oberhalb 31°C im Druckbereich von 120 bis 180 bar beschrieben. Die Entcoffeinierung auf diese Weise dauert 5 bis 15 Stunden und die anschliessende Trocknung 2 bis 5 Stunden.

In der DE—PS 22 12 281 wird die Entcoffeinierung von Rohkaffee mit flüssigem, also unterkritischem $CO_2$ bei Temperaturen unterhalb 31°C im Druckbereich von etwa 80 bis 400 bar beschrieben. Die Minimalzeit für die Entcoffeinierung auf diese Weise liegt bei 14 Stunden.

Neben der Entcoffeinierung von Rohkaffe wurden auch Versuche unternommen, Röstkaffee vom Coffein zu befreien. So beschreibt die DE—PS 21 19 678 die Entcoffeinierung von gemahlenem Röstkaffee mit überkritischem $CO_2$, wobei in einer ersten Stufe das Aromaöl abgetrennt und dann in einer Zweiten Stufe nach Anfeuchten des Röstkaffees die Entcoffeinierung durchgeführt wird. Durch dieses Vorgehen wird verhindert, dass Aromastoffe miteliminiert werden. Dieses zweistufige Verfahren dauert durchschnittlich 10 Stunden, d.h. pro Stufe je 5 Stunden.

Die DE—OS 26 38 383 beschreibt die Entcoffeinierung von Roh- oder Röstkaffee-Extrakten mit flüssigem oder überkritischem $CO_2$. Die Entcoffeinierung von Röstkaffee erfolgt derart, dass dieser zunächst bei erhöhter Temperatur mit Wasser behandelt wird, dann die Aromastoffe aus der wässrigen Lösung entfernt werden und dann das Coffein aus der wässrigen Lösung bei 15°C mit flüssigem Kohlendioxid oder mit überkritischem Kohlendioxid abgetrennt und aus der Kohlendioxidlösung gewonnen wird.

Den beiden zuletzt genannten Verfahren, die sich mit der Entcoffeinierung von Röstkaffee befassen, haftet der gemeinsame Nachteil an, dass sie zweistufig sind, d.h., das zunächst die Aromastoffe entfernt werden müssen und erst dann die eigentliche Entcoffeinierung erfolgen kann. Dadurch ist der Zeitaufwand für das Gesamtverfahren erheblich.

Allen bekannten Entcoffeinierungsverfahren, die unter Verwendung von Kohlendioxid arbeiten, ist gemeinsam, dass die Entcoffeinierungsstufe mindestens 4 bis 5 Stunden, in vielen Fällen erheblich länger dauert.

Darüber hinaus wird in den Fällen, in denen mit überkritischem $CO_2$ gearbeitet wird, eine Temperatur von in der Regel 40 bis 80° verwendet. Zur Schonung der Aromaeigenschaften des Produkts wäre es günstiger, wenn man bei niedrigeren Temperaturen arbeiten könnte.

Aufgabe der Erfindung ist es, ein Verfahren zur Entcoffeinierung von Röstkaffee zur Verfügung zu stellen, mit dem es möglich ist,

1. das Coffein auf schonende Weise von dem Röstkaffee abzutrennen,
2. einen hohen Entcoffeinierungsgrad zu erreichen,
3. die Entcoffeinierung einstufig durchzuführen, und
4. die Entcoffeinierung in möglichst kurzer Zeit durchzuführen.

Gelöst wird diese Aufgabe durch ein Verfahren der eingangs genannten Art, welches dadurch gekennzeichnet ist, dass die Extraktion mit flüssigem $CO_2$ bei einer Temperatur von 5° bis 31°C durchgeführt wird, wobei die Dauer der Extraktion so kurz gehalten wird, daß der Entcoffeinierungsfaktor

$$k = -\frac{\ln c_t/c_o}{t}$$

oberhalb 1 liegt, wobei $c_t$ den Coffeingehalt nach der Zeit t in %, $c_o$ den Anfangscoffeingehalt in % und t die Entcoffeinierungszeit in Stunden bedeuten. Vorzugsweise wird das Verfahren bei einer Temperatur von 15° bis 31°C durchgeführt. Der Druck liegt vorzugsweise bei 80 bis 150 bar.

Überraschenderweise hat sich herausgestellt, dass es mit dem vorliegenden Verfahren möglich ist, die Entcoffeinierung in vergleichsweise sehr kurzer Zeit, d. h. in weniger als 4 Stunden, in den meisten Fällen weniger als 3 Stunden, durchzuführen und dabei gleichzeitig einen ungewöhnlich hohen Entcoffeinierungsgrad zu erreichen. Dieses Ergebnis ist umso überraschender, als bei Anwendung der sonst üblichen Entcoffeinierungszeiten keine Verbesserung sondern eine Verschlechterung der Ergebnisse eintritt.

# EP 0 118 019 B2

Die Effektivität eines Entcoffeinierungsverfahrens kann mit Hilfe der folgenden Beziehung, in welche der Entcoffeinierungsgrad und die Entcoffeinierungszeit eingehen, ermittelt werden:

$$C_t = c_o \cdot e^{-kt}$$

Die Auflösung nach k ergibt:

$$k = -\frac{\ln c_t/c_o}{t}$$

Es bedeuten dabei $c_t$ den Coffeingehalt nach der Zeit t in %, $c_o$ den Anfangscoffeingehalt in % und t die Entcoffeinierungszeit in Stunden. Die Effektivität der Entcoffeinierung kommt in dem Entcoffeinierungsfaktor k zum Ausdruck.

Während mit allen bekannten Entcoffeinierungsverfahren, die unter Verwendung von überkritischem oder flüssigem $CO_2$ arbeiten, jeweils nur k-Werte von unterhalb 1, teilweise sogar nur Werte, die ganz beträchtlich unterhalb 1 liegen, erreicht werden, ist es mit dem erfindungsgemässen Verfahren möglich, k-Werte zu erreichen, die ganz deutlich über 1 liegen. Überraschenderweise ist das erfindungsgemässe Verfahren das effektivste Entcoffeinierungsverfahren, das bislang gefunden worden ist, gleichgültig ob mit überkritischem oder unterkritischem $CO_2$, bei hohen oder tiefen Temperaturen oder bei hohen oder niedrigen Drücken gearbeitet worden ist, oder ob zur Steigerung der Effektivität sogenannte Schleppmittel zum $CO_2$ zugesetzt worden sind, wie es beispielsweise in der DE—OS 27 37 793 beschreiben wird.

Das erfindungsgemässe Verfahren wird so durchgeführt, dass der Röstkaffee zunächst mit Wasser angefeuchtet wird. Dann wird das flüssige Kohlendioxid im Kreislauf durch den den Röstkaffee enthaltenden Arbeitsbehälter hindurchgeführt. Die Temperatur liegt zwischen 5° bzw. 15° und 30°C, vorzugsweise wird Umgebungstemperatur angewandt. Der Druck ist mindestens gleich dem Gleichgewichtsdruck des flüssigen $CO_2$ bei herrschender Temperatur, d. h. er beträgt zwischen 50 und 80 bar. Die Obergrenze liegt bei etwa 200 bar, wobei vorzugsweise 150 bar nicht überschritten werden sollten. Unter diesen Bedingungen beträgt die Dichte des flüssigen $CO_2$ etwa 0,7 bis 0,85.

Im Kreislaufsystem befindet sich ein Ionenaustauscher, der das Coffein selektiv aus dem flüssigen $CO_2$ entfernt. Da durch das flüssige $CO_2$ auch Aromastoffe aus dem Röstkaffee herausgelöst werden können, ist es erforderlich, dass der Ionenaustauscher hinsichtlich des Coffeins selektiv arbeitet. Als Ionenaustauscher können saure Ionenaustauscher, insbesondere stark saure Ionenaustauscher herangezogen werden. Beispielsweise können folgende Ionenaustauscher für den vorliegenden Zweck eingesetzt werden:

— Polystyrolharz mit Sulfonsäure als Ankergruppen,
— Polykondensationsharz mit Phenolsulfonsäure als Ankergruppe,
— Polyacrylat mit Carbonsäure als Ankergruppe.

Da bei Drücken oberhalb 150 und insbesondere oberhalb 200 bar zunehmend mehr Aromastoffe aus dem Produkt herausgelöst werden, ist es günstiger, unterhalb von einem Druck von 150 bar zu arbeiten.

Die Extraktion ist in der Regel nach 2 bis 3 Stunden beendet. Durch längere Extraktionszeiten lässt sich keine Erhöhung des Entcoffeinierungsgrades erzielen. Vielmehr nimmt der Entcoffeinierungsgrad, wie später noch gezeigt werden wird, überraschenderweise ab.

Nach Beendigung der Entcoffeinierung werden die im $CO_2$ enthaltenen Anteile an Aromastoffen durch einfaches Abdampfen des $CO_2$ isoliert und dem Röstkaffee wieder zugeschlagen. Das $CO_2$ wird zurückgewonnen. Die Reinigung der Ionenaustauscher bzw. die Wiedergewinnung des Coffeins kann dadurch erfolgen, dass die Ionenaustauscher mit flüssigem Kohlendioxid bei erhöhten Drücken, d. h. bei Drücken von oberhalb 200 bar, behandelt werden.

Bei einer bevorzugten Ausführungsform des Verfahrens ist das eingesetzte flüssige $CO_2$ bereits mit denjenigen Aromastoffen des Produktes vorbeladen, die dazu neigen, bei der Entcoffeinierung zu einem gewissen Anteil mitextrahiert zu werden, so dass durch die Vorbeladung diese Tendenz deutlich verringert wird. Die Vorbeladung erfolgt derart, dass man trockenes flüssiges $CO_2$ über Röstkaffee leitet und die Aromastoffe entzieht. Das auf diese Weise mit Röstkaffee-Aromastoffen vorbeladene, vorzugsweise gesättigte, $CO_2$ entzieht dann — angefeuchtet — selektiver das Coffein aus einer neuen Röstkaffeecharge.

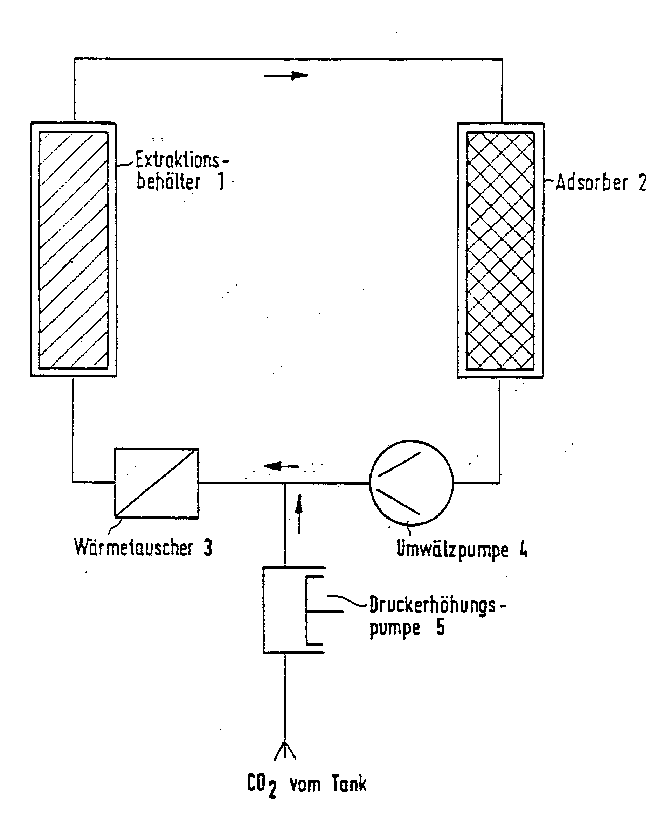

Die Figur zeigt in schematischer Darstellung eine Vorrichtung, mit der das erfindungsgemässe Verfahren durchführbar ist. Die Figur zeigt einen Extraktionsbehälter 1 und einen Adsorber 2, die über einen Wärmeaustauscher 3 und eine Umwälzpumpe 4 im Kreislauf geschaltet sind. Über die Druckerhöhungspumpe 5 wird $CO_2$ von einem Tank (nicht dargestellt) in dem Kreislauf eingespeist. Der Wärmeaustauscher 3 dient zur Anpassung an die Arbeitstemperatur. Im Extraktionsbehälter 1 befindet sich der gemahlene Röstkaffee, während im Adsorber 2 der Ionenaustauscher untergebracht ist. Die Umwälzpumpe 4 besorgt den Kreislaufbetrieb.

Die Erfindung wird dadurch die nachfolgenden Beispiele weiter erläutert.


Beispiel 1

1,5 kg gemahlener Röstkaffee mit einer durchschnittlichen Korngrösse von 1 mm wurden mit 0,5 kg Wasser angefeuchtet. Im Arbeitsbehälter oder Extraktionsbehälter 1 wird er bei 29°C, 2,5 Stunden lang mit flüssiger Kohlensäure bei einem Druck von 85 bar (Dichte des $CO_2$: 0,703) extrahiert. Als Adsorptionsmittel wurde ein stark saurer Ionenaustauscher eingesetzt. Der Ausgangscoffeingehalt des Kaffees betrug 1,74%. Nach der Extraktion wurden die im $CO_2$ enthaltenen Aromastoffe durch Abdampfen des $CO_2$ isoliert und dem Kaffee wieder zugeschlagen. Der fertig bearbeitete Kaffee hatte einen Restcoffeingehalt von 0,028%. Daraus errechnet sich der Entcoffeinierungsfaktor k zu k = 1,65. Der fertige Röstkaffee stellte ein einwandfreies Produkt dar, welches sich sensorisch vom Ausgangskaffee praktisch nicht unterscheiden liess.

Beispiele 2 bis 6

Beispiel 1 wurde wiederholt, wobei unterschiedliche Drücke und Entcoffeinierungszeiten angewandt wurden. Die Entcoffeinierungstemperatur betrug jeweils 29°C. Die Ergebnisse sind in der nachfolgenden Tabelle I zusammengestellt.

Tabelle I

| Bei-spiel | Druck (bar) | Entcoffei-nierungs-zeit (Stunden) | Anfangs-coffein-gehalt $c_0$ (%) | End-coffein-gehalt $c_t$ (%) | Entcoffei-nierungs-grad (%) | Entcoffei-nierungs-faktor k |
|---|---|---|---|---|---|---|
| 2 | 300 | 4,3 | 1,74 | 0,17 | 90,2 | 0,54 |
| 3 | 200 | 4,3 | 1,74 | 0,17 | 90,2 | 0,54 |
| 4 | 200 | 2,0 | 1,74 | 0,059 | 96,6 | 1,35 |
| 5 | 85 | 2,5 | 1,74 | 0,030 | 98,3 | 1,62 |
| 6 | 85 | 2,5 | 1,74 | 0,032 | 98,2 | 1,60 |

Die Tabelle I zeigt, dass die besten Ergebnisse bei etwa Gleichgewichtsdruck und einer Entcoffeinierungszeit von etwa 2,5 Stunden erzielt werden. Weiterhin zeigt die Tabelle, dass bei zu hohem Druck und zu langen Entcoffeinierungszeiten schlechtere Entcoffeinierungsgrade erzielt werden. Dies könnte darauf zurückzuführen sein, dass bei höheren Drücken und insbesondere bei längeren Entcoffeinierungszeiten als 4 Stunden eine Desorption des Coffeins von der Austauschersäule erfolgt. Überraschenderweise werden also die besten Ergebnisse bei vergleichsweise sehr kurzen Entcoffeinierungszeiten erreicht. Bei den erfindungsgemässen Beispielen 4 bis 6 liegt der Wert des Entcoffeinierungsfaktors k sehr deutlich über dem Wert 1,0. Im Vergleich hierzu zeigt die nachfolgende Tabelle II, dass bei bekannten Entcoffeinierungsverfahren unter Verwendung von Kohlendioxid k-Werte erreicht werden, die immer unter 1,0, zum Teil erheblich unter 1,0, liegen.

# EP 0 118 019 B2

### Tabelle II

| Patent | Temperatur ($°C$) | Druck (bar) | Entcoffeinierungszeit (Stunden) | Endcoffeingehalt $c_t$ (%) | Ausgangscoffeingehalt $c_o$ (%) | Entcoffeinierungsfaktor k |
|---|---|---|---|---|---|---|
| DE-PS 20 05 293 | 70 | 160 | 5 – 30 | 0,01 | 1,2 | 0,95 – 0,15 |
| DE-PS 21 19 678 | 50 | 280 | 4 | 0,03 | 1,2 | 0,922 |
| DE-PS 22 12 281 | 20 | 350 | 14 | 0,05 | 1,04 | 0,22 |
| DE-PS 27 27 191 | 70 | 250 | 8 | 0,08 | 1,02 | 0,322 |

## Patentansprüche

1. Verfahren zum Extrahieren von Coffein aus gemahlenem Röstkaffee mit $CO_2$ bei einem Druck von 50 bis 200 bar, wobei das mit Coffein beladene $CO_2$ zur Abtrennung des Coffeins durch einen sauren Ionenaustauscher geführt wird, dadurch gekennzeichnet, daß die Extraktion mit flüssigem $CO_2$ bei einer Temperatur von 5° bis 31°C durchgeführt wird, wobei die Dauer der Extraktion so kurz gehalten wird, daß der Entcoffeinierungsfaktor

$$k = - \frac{\ln c_t/c_o}{t}$$

oberhalb 1 liegt, wobei $c_t$ den Coffeingehalt nach der Zeit t in %, $c_o$ den Anfangscoffeingehalt in % und t die Entcoffeinierungszeit in Stunden bedeuten.

2. Verfahren nach Anspruch 1, dadurch gekennzeichnet, dass der Druck 80 bis 150 bar beträgt.

## Revendications

1. Procédé d'extraction de la caféine à partir du café torréfié moulu à l'aide de $CO_2$ sous une pression de 50 à 200 bars, le $CO_2$ chargé de caféine passant à travers un échangeur d'ions acide pour la séparation de la caféine, caractérisé en ce qu'on conduit l'extraction avec du $CO_2$ liquide à une température de 5 à 31°C et, en ce que la durée de l'extraction étant maintenue suffisamment courte pour que le degré de décaféination:

$$k = - \frac{\log_e c_t/c_o}{t}$$

reste supérieur à 1, $c_t$ étant la teneur en caféine au bout du temps t en %, $c_o$ étant la teneur en caféine initiale en % et t la durée de décaféination en heures.

2. Procédé selon la revendication 1, caractérisé en ce que la pression va de 80 à 150 bars.

## Claims

1. Process for extracting caffeine from ground roasted coffee with $CO_2$ at a pressure of 50 to 200 bar,

5

the $CO_2$ loaded with caffeine being passed through an acidic ion exchanger for separating off the caffeine, characterised in that the extraction is carried out with liquid $CO_2$ at a temperature from 5° to 31°C, the duration of the extraction being kept so short that the decaffeination factor

$$k = -\frac{\ln c_t/c_o}{t}$$

is above 1, $c_t$ being the caffeine content after the time t in %, $c_o$ the initial caffeine content in % and t the decaffeination time in hours.

2. Process according to claim 1 characterised in that the pressure is 80 to 150 bar.

Extraktionsbehälter 1

Adsorber 2

Wärmetauscher 3

Umwälzpumpe 4

Druckerhöhungspumpe 5

CO$_2$ vom Tank